# EUROPEAN PATENT SPECIFICATION

(11) **EP 0 455 735 B1**
(45) Date of publication and mention of the grant of the patent: **14.09.1994**
(21) Application number: 90903466.2
(22) Date of filing: 25.01.1990
(51) Int. Cl.: G01N 33/543, G01N 33/53, G01N 33/548, G01N 33/566, G01N 33/58

(54) **AVIDIN-BIOTIN ASSISTED IMMUNOASSAY**
AVIDIN-BIOTIN ASSISTIERTES IMMUNASSAYVERFAHREN
IMMUNOANALYSE ASSISTEE PAR AVIDINE-BIOTINE

(30) Priority: 30.01.1989 US 302877
(43) Date of publication of application: 13.11.1991
(73) Proprietor: EPITOPE, INC., Beaverton Oregon 97006 (US)
(72) Inventor: THIEME, Thomas, Independence, OR 97351 (US); FERRO, Adolph, Lake Oswego, OR 97035 (US); FELLMAN, Jack, H., Portland, OR 97219 (US); GAVOJDEA, Stefan, Beaverton, OR 97006 (US)
(74) Representative: Clisci, Serge
(86) International application number: US9000378
(87) International publication number: WO9008957

(56) References cited:
- EP-A- 21 214
- EP-A- 60 700
- EP-A- 71 976
- WO-A-86/03589
- GB-A- 2 109 931
- US-A- 4 228 237
- US-A- 4 535 057
- DIALOG INFORMATION SERVICES, Medline file 154; A. PLEBANI et al., acc. no. 05317155#
- DIALOG INFORMATION SERVICES, Medline file 154; T. TAKATORI et al., acc. no. 05948028#
- PATENT ABSTRACTS OF JAPAN, vol. 4, no. 144 (C-27), 14 July 1980#

## Description

### BACKGROUND OF THE INVENTION

This invention relates to the detection for screening and diagnostic purposes of antibodies in body fluids such as saliva, urine, tears, serum or plasma. In particular, this invention relates to the detection of antibodies which are present in low amounts in such fluids or where small amounts of such fluids are available and yet the antibodies present have antigenic specificities characteristics of particular disease states, and are of diagnostic value.

Body fluids of mammals, such as serum, plasma, saliva, tears, urine, milk, seminal fluid, synovial fluid, etc. can contain antibodies which are useful in the diagnosis of diseases, including those of bacterial and viral infection and of autoimmune origin. Body fluids, such as saliva, have significant advantage over serum and plasma as sources of these diagnostically valuable antibodies since they can be obtained without the facilities and hazards attendant with the taking of blood samples. However, often the concentration of antibodies is so low in these fluids as to make conventional tests for antibodies impractical.

Saliva, in particular, presents problems as a diagnostic indicator. These problems stem from the low concentration of antibodies in saliva and the inconvenience of collecting and processing quantities of saliva sufficient for a reliable diagnostic test, which can be quickly performed.

### PRIOR ART

GB-A-2 109 931 discloses an immunoassay system in which the filtrate from a column is analysed by means of a competitive assay.

WO-A-86/03589 discloses an immunoassay utilising a column-like apparatus, trapping analytes by means of an immobilised binding agent specific for the analyte of interest.

EP-A-0 060 700 discloses a competitive immunoassay system where the analyte of interest is trapped on a solid support by a biospecific agent. The avidin-biotin system is indicated as a possible label.

US-A-4 228 237, EP-A-0 071 976 and US-A-4 535 057 describe the use of specific binding partner-biotin and avidin-label conjugates as a signal detection system in immunoassays.

### SUMMARY OF THE INVENTION

In one aspect, the present invention is a method for detection of an antibody of interest in a body fluid containing said antibody of interest and other antibodies comprising the steps of providing an antibody-binding protein covalently bound to a support; contacting and incubating a test body fluid containing said antibody of interest and other antibodies with said protein-bound support whereby said protein reacts with said antibodies to immobilize said antibodies ; including said antibody of interest contacting and incubating a biotinylated antigen specific to said antibody of interest with the immobilized antibody of interest whereby said biotinylated antigen reacts with said immobilized antibody to immobilize said biotinylated antigen; contacting and incubating a solution of avidin, said avidin covalently linked to an enzyme, with the immobilized biotinylated antigen-immobilized antibody-protein bound complex whereby said enzyme-labelled avidin reacts with said complex to become immobilized; contacting and incubating said immobilized complex with a substrate solution wherein said enzyme linked to avidin induces a reaction of said substrate and produces a detectable reaction product; and correlating said detectable reaction product to the presence of said antibody of interest to be detected.

In another aspect the invention is a method for detection of antibody of interest in a body fluid containing said antibody and other antibodies, comprising the steps of providing an antibody-binding protein covalently bound to a support; contacting and incubating a test body fluid containing said antibody and other antibodies with a solution of an antigen specific for the antibody of interest, said antigen being biotinylated; contacting and incubating said combined solution with a solution of avidin, said avidin covalently linked to an enzyme; contacting and incubating said combined solution with the support such that a complex of antibody-biotinylated antigen- enzyme labelled avidin is immobilized by said support; contacting and incubating said immobilized complex with a substrate solution wherein said enzyme linked to avidin induces a reaction of said substrate and produces a detectable reaction product; and correlating said detectable reaction product to the presence of said antibody to be detected.

A further aspect of the invention is a method for detecting an antigen in a test biological fluid containing said antigen, comprising the steps of providing an Fc receptor antibody-binding protein covalently bound to a support; providing a solution of test fluid containing said antigen and containing a small amount of antibody against said antigen in aqueous buffer; contacting and incubating said solution with said support such that a complex antibody and antigen is immobilized by said support; providing a solution of monoclonal antibody modified so that it is a Fab fragment, lacking the Fc portion which binds to the protein but retaining its ability to bind antigen, said antibody being biotinylated; contacting and incubating said solution with the immobilized complex such that an antibody-antigen-Fab antibody complex is immobilized by said support; providing a solution of avidin, said avidin covalently linked to an enzyme; contacting and incubating said solution with the immobilized antibody-antigen-Fab antibody complex whereby said enzyme-labelled avidin reacts with said complex to immobilize said enzyme-labelled avidin; contacting and incubating said immobilized complex with a substrate solution wherein said enzyme linked to avidin induces a reaction of said substrate and produces a detectable reaction product on the immobilised complex; and correlating said detectable reaction product to the presence of said antibody to be detected. The invention also contemplates a test kit comprising a support having covalently bound thereto an antibody-binding protein. The kit also comprises a biotinylated antigen specific to an antibody of interest (for detection of antibody), or a biotinylated monoclonal antibody modified to be a Fab fragment, lacking the Fc portion which binds to the protein but retaining ability to bind to an antigen of interest (for detection of antigen), enzyme-linked avidin solution and enzyme substrate.

### BRIEF DESCRIPTION OF THE DRAWINGS

Figure 1 schematically illustrates the binding mechanism utilized by the present invention; and
Figure 2 diagrammatically represents the apparatus used in the present invention.

### DESCRIPTION OF THE PREFERRED EMBODIMENT

A preferred embodiment of the present invention comprises a method of rapidly isolating antibodies from a body fluid by passing the fluid through a porous matrix to which is chemically bonded the substance protein A, a protein which rapidly binds to antibody molecules. The antibodies are thus rapidly removed from the fluid as it filters through the matrix and the antibodies are concentrated in the matrix for the subsequent colorimetric assay for antigenic specificity. It is preferred that the porous matrix is a predetermined amount of a beaded agarose gel with covalently bonded protein A which is placed in a column made of transparent material. The column has a porous frit at the bottom to retain the agarose gel while allowing the body fluid to quickly filter through the gel. The amount of protein A-containing gel in the column is sufficient to bind an optimal amount of the antibodies in the body fluid and provide an adequate size surface for the subsequent colorimetric assay.

Protein A is a protein isolated from the cell wall of the bacteria staphylococcus aureus (Cowan strain 1). Protein A (also called Type 1 Fc receptor) is available commercially as a protein isolated from this bacteria and also in a recombinant form expressed in other bacteria. Protein A can be covalently linked to agarose by any of several commonly used chemical reactions. Some examples of these techniques are the following: Amino groups or carboxyl groups are introduced into the agarose matrix by the cyanogen bromide method with diamino alkanes (introduction of amino group) or amino carboxylic alkanes (introduction of carboxyl groups). This method is described by M. Wilchek et al., "The purification of biologically active compounds by affinity chromatography," Methods of Biochemical Analysis, 23:347-385, 1986. These derivatized agarose gels are then coupled to carboxyl groups or amino groups on the protein by the use of the commonly used carbodiimide chemistries or the use of such commonly used leaving groups as N-hydroxysuccinimide which is introduced into the amino derivatized agarose with O-bromoacetyl-N-hydroxysuccinimide. Other known techniques can be used to covalently bind protein A to agarose or other polysaccharide matrices.

Other proteins which rapidly and avidly bind antibodies are available, and can be used instead of protein A for the purposes described in this application. These proteins include certain proteins isolated from group A streptococci (Type II Fc receptor), protein G (Type III Fc receptor) isolated from most human C and G streptococcus strains, Type IV Fc receptor isolated from some strain G streptococcus strains, and Types V, VI Fc receptors isolated from streptococcus zooepidimicus. Each of these proteins has certain advantages over the others in its strength of binding to different subclasses of immunoglobulins and immunoglobulins from different mammalian species.

Other types of porous matrices are suitable for the purposes of this invention if they have low non-specific binding of proteins and functional groups such as hydroxyl groups or unique chemistries which allow the covalent binding of protein A or other proteins which bind antibodies. An example of such matrix is the methacrylamide, glycidyl methacrylate beaded polymer available from Röhm Pharma under the name "Eupergit C".

After binding of antibodies to the porous matrix as indicated above, excess amounts of the body fluid are removed from the matrix by washing with a suitable aqueous buffer. The matrix with bound antibody is then used as a substrate to assay for the antigenic specificity of the bound antibody.

Several methods are available for the assay of the antigenic specificity of isolated antibodies. These include the use of enzymes such as horseradish peroxidase or alkaline phosphatase covalently linked to the antigens of interest. This technique is illustrated in T. Kitagawa et al., "Enzyme coupled immunoassay of insulin using a novel coupling reagent," Journal of Biochemistry, 79:233-236, 1976. These enzymes are used to develop color reactions which indicate the presence of the antigen. Another method which is available is the covalent binding of biotin to the antigen as in G.R. Dreesman et al., U.S. Patent 4,535,057; D.A. Fuccillo, "Application of the avidin-biotin technique in microbiology," Bio-Techniques, 3:494-501, 1985; and M. Wilchek et al., "The use of the avidin-biotin complex as a tool in molecular biology," Methods of Biochemical Analysis, 26:1-45. Biotin (also known as Vitamin H) has the following chemical structure:

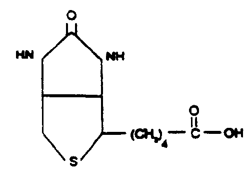

It is bound with very high avidity (Kd 10-15M) by the protein avidin. Four biotin molecules are bound per avidin molecule. When enzymes such as horseradish peroxidase or alkaline phosphatase are chemically linked to the avidin molecule, the avidin-biotin interaction can constitute a molecular bridge between the antigen of interest and the enzyme which is used to develop the color reaction that indicates the presence of the antigen. Because each avidin molecule has four biotin binding sites, the number of enzyme molecules per antibody molecule is increased in the presence of excess biotinylated antigen and the sensitivity of the assay is increased.

The chemical bond between the antigen and biotin molecule can be formed by a number of different chemical reaction sequences which are available. These reactions typically utilize N-hydroxysuccinimidyl or iodo leaving groups to derivative amino groups in the protein structures of the antigen. Typical reagents are shown below:

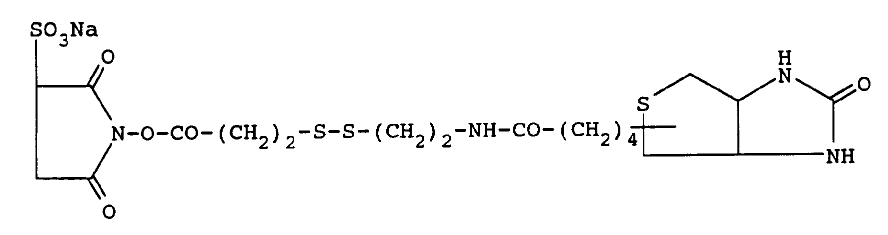

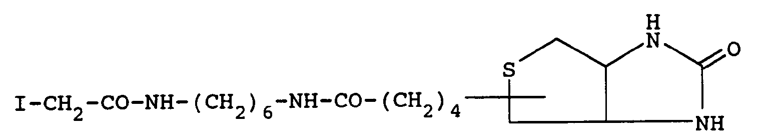

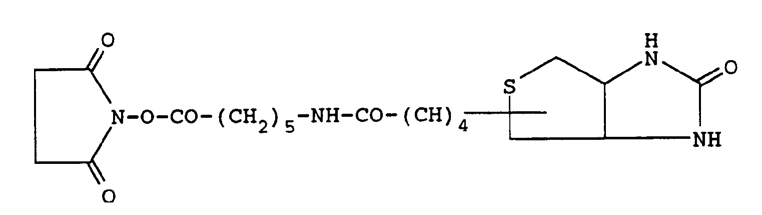

Carbohydrate antigens can be biotinylated by the reaction of biotin hydrazide with aldehyde groups produced in polysaccharide structures after reaction with periodate. The selection of the best biotinylation system among these and other available chemical reactions depends on the particular nature of the antigen being tested.

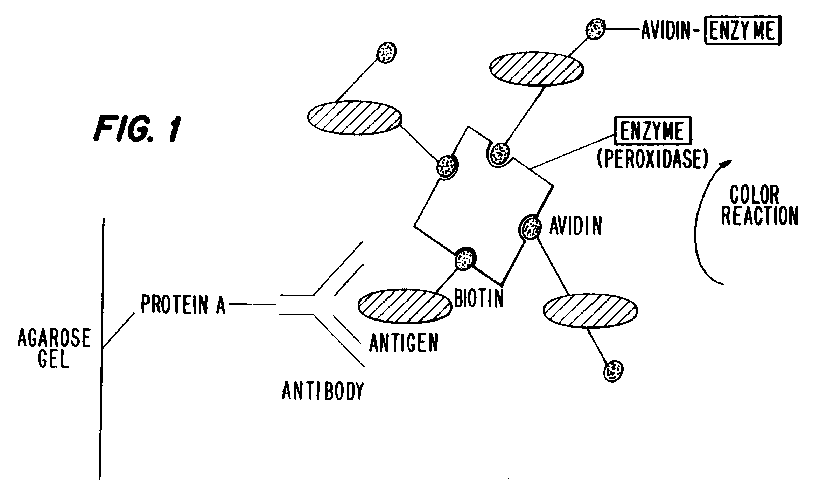

The present invention represents a unique application of the use of the biotin-avidin reaction to link antigen with an enzyme detection system. The unique use lies in the fact that the antigen has been immobilized as it passes through a porous matrix containing specific antibody which has in turn been immobilized by protein A as shown in Figure 1 which illustrates schematically the system.

In the presence of excess biotinylated antigen, many avidin-enzyme conjugates can bind to the gel for every antibody immobilized by protein A. This gives the technique its high sensitivity.

The enzyme coupled to the avidin determines the colorimetric assay used in the detection of antigen, which ultimately is a test for the presence of certain types of antibody in the biological fluid. Two of the more commonly used enzymes are alkaline phosphatase and horseradish peroxidase. These enzymes may be chemically bonded and linked to the avidin molecule utilizing commonly used chemical pathways for protein; protein covalent linkage includes the use of glutaraldehyde or the use of other homobifunctional and heterobifunctional reagents such as disuccinimidyl suberate and succinimidyl-N-(4-carboxycyclohexylmethyl)-maleimide (S. Yoshitake et al., "Conjugation of glucose oxidase from Aspergillus niger and rabbit antibodies using N-hydroxy-succinimide ester of N-(4-carboxy-cyclohexylmethyl)-maleimide," Eur. J. Biochem, 101:395-399). These chemistries utilize the reactivity of the amino group of proteins with the succinimidyl ester, and the reactivity of the free SH group of proteins with the maleimide moiety.

If horseradish peroxidase is used as the detection enzyme, typical substrates would be diaminobenzidine, 4-chloro-1-naphthol, or 3,3′,5,5′-tetramethylbenzidine(7). When any of these materials is mixed with hydrogen peroxide and exposed to the enzyme, a darkly colored polymeric material is formed. This colored product is fast, i.e., insoluble and precipitating on the solid support. The production of this colored precipitate indicates the presence of the enzyme and in the context of this invention the presence of antibodies specific for the antigen being investigated. Other materials producing highly colored precipitates are used to indicate the presence of alkaline phosphatase such as a mixture of 4-chloro-2-methylaniline and 3-hydroxy-2-naphthoic acid 4′ chloro-2′ methyl anilide phosphate, or a mixture of 5-bromo-4-chloro-3-indolyl-phosphate and nitroblue tetrazolium.

In the present invention, the capture of antibodies by the Protein A attached to the insoluble matrix occurs in minutes with a total assay time of less than 15 minutes. The use of the column technique also lends speed and convenience to the present invention, and permits the concentration of antibodies from a large volume of fluid.

### EXAMPLE I

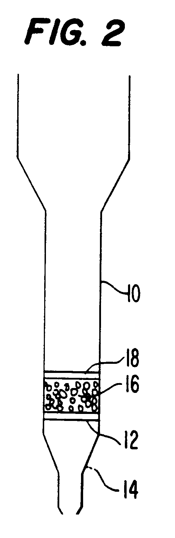

The following is a typical and practical use of the ideas and techniques which are the basis of this invention: Crosslinked agarose consisting of beads (75-300 µm (micron) diameter) to which protein A has been linked by the techniques indicated above (either by the producer or a commercial supplier, e.g., Bio Rad Corp. Product 1536154) is washed in phosphate buffered saline (PBS) and incubated for 12 hours at 4°C as a 25% suspension in PBS containing 0.75% gelatin, 0.3% Tween™20 detergent. This reduces non-specific binding of proteins to the agarose. The agarose-protein A beads are again washed in PBS. As shown in Figure 2, a clean polystyrene column 10 (8 mm I.D. x 102 mm) with a volume of 6 milliliters and a porous (70 µm pore size) polyethylene disk (frit) 12 at the bottom is used (e.g., Pierce Chemical Co. product 29920). A cap 14 is used on the bottom tip of the column to control the flow of liquid through the column. Columns of other dimensions which fulfill the basic requirements of the invention are usable. Two hundred microliters of the prepared agarose-protein A beads 16 are placed on the frit at the bottom of the column with adequate PBS for transfer. A second frit 18 is placed on top of the settled agarose beads resulting in a 4 mm thick disk of agarose beads sandwiched between 2 porous frits. The column described here fulfills the essential requirement of a device which will (1) contain an adequate quantity of agarose-protein A beads, (2) allow easy observation of the beads, (3) allow the flow of a body fluid to be tested through the beads with adequate contact surface between solutes in the body fluid and the protein A attached to the beads, (4) allow the sequential addition and washing out of the reagents used to perform the colorimetric tests of the antibodies in the body fluid. After placing the agarose-protein A beads in the column as described, about one milliliter of the body fluid to be tested is diluted with 2 milliliters of an aqueous buffer which is 0.25M in TRIS™ (Tris (hydroxy methyl amino methane)), 0.37% gelatin, 0.30% Tween™20, 0.075M NaCl, and 0.01M in sodium phosphate. This buffer is called "diluent buffer" and has a pH of about 8.0. The diluted body fluid is placed in the column on the top of the upper frit and allowed to drain through the column by removing the bottom cap (3 to 5 minutes for this step). The column is then washed twice with 3 ml. portions of diluent buffer (3 minutes per wash). Ten micrograms of biotinylated antigen (prepared by producer) is added to 2 ml. of diluent buffer and drained through the column (2 minutes). Additional sensitivity can be obtained by preincubating antigen with body fluid for 5 minutes and then draining through the column. This is followed by 2 x 3 ml. washes with diluent buffer (3 minutes each). Twenty micrograms of avidin-peroxidase conjugate (prepared by producer or commercial supplier, e.g., Sigma Chemical Co. product 3151) is dissolved in 2 ml. diluent buffer and drained through the column (2 minutes). Again the column is washed 2 times with 3 ml. of diluent buffer (3 minutes each). These washes are followed by passing 2 ml. of a substrate solution through the column. The substrate solution can be 0.5 mg/ml. of 3,3 diaminobenzidine in PBS containing 0.00004 percent H₂O₂ (2 minutes). The extent of darkening of the gel at this point indicates the presence of antibodies in the biological fluid specific for the test antigen. An additional wash with water allows the test column to be capped and stored at refrigerator temperature with stable color development for several days.

Controls consist of columns run at the same time with known positive and negative samples of body fluids or suitable solutions of positive and negative antibodies. Alternative or additional controls for the purpose of color comparison can be agarose without protein A (negative) or agarose with biotin coupled to it. Biotin, which contains a carboxylic acid, can be coupled to agarose by the same chemical reactions used to couple protein A to agarose. These color-comparison-controls can be run in the same column as the test by simply placing the additional aliquots of agarose in the column with the agarose-protein A; each agarose aliquot is separated from the others by a polyethylene frit.

### EXAMPLE II

The procedure described in Example I for assaying 1 ml. of body fluid takes about 27 minutes. This time can be significantly reduced by the following modification: One milliliter of body fluid is combined with 2 ml. of diluent buffer. Ten micrograms of biotin-antigen complex are added followed by incubation for 2 minutes at room temperature. Twenty micrograms of avidin-peroxidase complex are added and the mixture is immediately passed through the column (3 minutes). This is followed by 2 washes with 3 ml. of diluent buffer (6 minutes) and color development with 2 ml. of diaminobenzidine-H₂O₂ substrate solution (2 minutes). The total assay time with this modified procedure is about 13 minutes and the sensitivity is equal to or greater than that described earlier.

### EXAMPLE III

Using the abbreviated column procedure of Example II, we have tested the saliva and serum of Acquired Immune Deficiency (AIDS) patients which are confirmed to have serum antibodies against the Human Immunodeficiency Virus-l (HIV-1) proteins (positive samples). Ten micrograms of biotinylated HIV viral lysate were used per column. Using 6 microliters of positive serum or 150 microliters of positive saliva, a near maximum dark brown color was obtained on the agarose beads using the diaminobenzidine substrate. Control serum and saliva from uninfected persons gave essentially colorless agarose beads with the same procedure. The same results have been obtained using as antigen an HIV protein produced by expression of a recombinant gene (commercial product, envelope peptide p121, Centocor, Inc.). Similar results have also been obtained using a viral lysate of the virus HTLV-1 and serum from infected patients.

In a like manner, this invention should be useful for the detection of disease specific antibody in body fluids of patients with infection by such agents as Hepatitis B and Herpes Simplex Type I and Type II.

Another format which fulfills the basic function of the column is to place the sandwich of frit-protein A-agarose-frit in a disposable plastic pipette tip. The frits wedge tightly against the walls of the tip, confining the agarose beads and allowing the flow of liquid through the beads. When attached to the standard hand pipetting unit, the tip can be used to draw the body fluids and various reagents and washing fluids through the agarose beads.

### EXAMPLE IV

In addition to its ability to detect antibodies specific for HIV and other antigens, the present invention can be modified to directly detect the antigens of interest. Biotinylated antigen is omitted in this procedure. The biological fluid to be tested is added to blocking buffer in the column. Two micrograms of human antibody against the antigen(s) is also added to this solution in the blocking buffer. After 5-10 minutes incubation, there is added 2 micrograms of a mouse monoclonal antibody that has been modified so that it is a Fab fragment (lacking the Fc part of the molecule which binds to Protein A, but retaining its ability to bind antigen). (D.W. Dresser, "Assays for immunoglobulin-secreting cells," in D.M. Weir (Ed.), Handbook of Experimental Immunology, 34d ed., Blackwell Scientific Publications, Oxford; and J.J. Langone, "Protein A of Staphylococcus aureus and related immunoglobulin receptors produced by Streptococci and Pneumonococci," Advances in Immunology, 32:157-252.) This modified monoclonal antibody is also biotinylated as previously described. The monoclonal antibody binds to those portions of the antigen which are not bound to the human antibody. After another 5-10 minutes incubation, the avidin-enzyme detection reagent is added and the mixture drained through the column. After washing, chromogenic detection is carried out as before. The monoclonal antibody Fab fragment will only bind to the column, and thus color development will only be seen, if the antigen of interest is present. In contrast to other immunoassays for the direct detection of antigen, this technique captures antigen as an antigen-antibody complex by binding of antibody, including the antibody endogenously present in the biological sample. Also, larger volumes of biological fluid can be concentrated on a small detection surface.

### Example V

This technique can also be adapted to biological fluids which lack endogenous antibodies against the antigen of interest and where there are no other sources of human antibody specific for the antigen. This would be the case, for example, in a plant tissue extract to be assayed for antigens of a plant pathogen. Here, two monoclonal antibodies specific for the antigens of the pathogen would be used. One antibody would be added to the extract to bind the antigen and mediate its binding to the immobilized Protein A. The second monoclonal would be made into a biotinylated Fab fragment and used as a detection antibody.

A specific potential application of this technique exists for the detection of Corynebacterium sepedonicum infection of potatoes. C. sepedonicum infection produces bacterial ring rot in potatoes, a disease of significant commercial impact. Stem and tuber extracts of affected plants contain many of these gram-positive bacteria. A number of monoclonal antibodies have been produced in mice to the antigens of C. sepedonicum which can be used in an immunoassay for the organism. Extracts of suspect plant tissues are prepared according to standard techniques in fluid containing detergent to maximize solubility of the bacterial antigens. Insoluble materials are removed by filtration and centrifugation. Between 1 and 5 mL of this extract are added to the column described above. The column contains 1 mL of diluent buffer and 10 micrograms of a monoclonal antibody against a principal bacterial antigen. Ten micrograms of a second monoclonal antibody, biotinylated and present as a Fab fragment, are also present. This monoclonal antibody is specific for a second epitope on the same bacterial antigen. After 5 minutes incubation, the mixture is drained through the column. The column is washed with diluent buffer as described above. Avidin linked to enzyme is then passed through the column followed by washes as previously described in Examples I and II. Suitable chromogenic substrates for the enzyme are added and color development estimated in comparison to positive and negative controls to assess the presence of bacteria in the original tissues.

## Claims

1. A method for detection of an antibody of interest in a body fluid containing said antibody of interest and other antibodies, comprising the steps of :
a. providing an antibody-binding protein covalently bound to a support ;
b. contacting and incubating a test body fluid containing said antibody of interest and other antibodies with said protein-bound support of step (a) whereby said protein reacts with said antibodies to immobilize said antibodies, including said antibody of interest ;
c. providing a solution of an antigen specific to said antibody of interest, said antigen being biotinylated ;
d. contacting and incubating said biotinylated antigen of step (c) with the immobilized antibody of interest of step (b) whereby said biotinylated antigen reacts with said immobilized antibody of interest to immobilize said biotinylated antigen ;
e. providing a solution of avidin, said avidin covalently linked to an enzyme ;
f. contacting and incubating said solution of step (e) with the immobilized biotinylated antigen-immobilized antibody-protein bound complex of step (d) whereby said enzyme-labelled avidin reacts with said complex to immobilize said enzyme-labelled avidin ;
g. contacting and incubating said immobilized complex of step (f) with a substrate solution wherein said enzyme linked to avidin induces a reaction with said substrate and produces a detectable reaction product on the immobilized complex ; and
h. correlating said detectable reaction product on the immobilized complex to the presence of said antibody of interest to be detected.

2. The method of claim 1, wherein said body fluid is saliva.

3. The method of claim 1, wherein said support is any porous material having low non-specific binding of proteins and functional groups or other chemistries which allow binding or proteins that bind antibodies.

4. The method of claim 3, wherein said support is agarose.

5. The method of claim 4, wherein color-comparison controls are run using additional aliquots of agarose separated from one another with polyethylene disks.

6. The method of claim 1, wherein said protein is protein A.

7. The method of claim 1, wherein said protein is derived from sources chosen from the group of Types II, III, IV, V and VI Fc receptor.

8. The method of claim 1, wherein after said test fluid is contacted and incubated with said protein-bound support, said support is washed.

9. The method of claim 1, wherein after said biotinylated antigen is contacted and incubated with said immobilized antibody, said support is washed.

10. The method of claim 1, wherein after said enzyme-linked avidin is contacted and incubated with said immobilized antigen complex, said support is washed.

11. The method of claim 1, wherein said support is trapped within a column which is open-ended.

12. The method of claim 11, wherein the column is polystyrene.

13. The method of claim 11, wherein the support is supported by a porous polyethylene disk.

14. The method of claim 1, wherein the test body fluid is diluted before application with an aqueous buffer.

15. A method for detection of an antibody of interest in a body fluid containing said antibody and other antibodies comprising the steps of :
a. providing an antibody-binding protein covalently bound to a support ;
b. contacting and incubating a test body fluid containing said antibody of interest and other antibodies with a solution of an antigen specific for the antibody of interest, said antigen being biotinylated ;
c. contacting and incubating said combined solution of step (b) with a solution of avidin, said avidin covalently linked to an enzyme ;
d. contacting and incubating said combined solution of step (c) with a support of step (a) such that a complex of antibody-biotinylated antigen-enzyme labelled avidin is immobilized by said support ;
e. contacting and incubating said immobilized complex of step (d) with a substrate solution wherein said enzyme linked to avidin induces a reaction with said substrate and produces a detectable reaction product on the immobilized complex ; and
f. correlating said detectable reaction product on the immobilized complex to the presence of said antibody to be detected.

16. The method of claim 15, wherein after said combined solution of step (c) is contacted and incubated with the support of step (a), said support is washed.

17. The method of claim 15, wherein the antigen is HIV-1.

18. The method of claim 1 or 15, wherein the assay is performed in a pipette tip.

19. A method for detecting an antigen in a test biological fluid containing said antigen, comprising the steps of :
a. providing an Fc receptor antibody-binding protein covalently bound to a support ;
b. providing a solution of test fluid containing said antigen and containing a small amount of antibody against said antigen in aqueous buffer;
c. contacting and incubating said solution of step (b) with said support of step (a) such that a complex of antibody and antigen is immobilized by said support ;
d. providing a solution of monoclonal antibody modified so that it is an Fab fragment, lacking the Fc portion which binds to the protein but retaining its ability to bind said antigen, said antibody being biotinylated ;
e. contacting and incubating said solution of step (d) with the immobilized complex of step (c) such that an antibody-antigen-Fab antibody complex is immobilized by said support ;
f. providing a solution of avidin, said avidin covalently linked to an enzyme ;
g. contacting and incubating said solution of step (f) with the immobilized antibody-antigen-Fab antibody complex of step (e) whereby said enzyme-labelled avidin reacts with said complex to immobilize said enzyme-labelled avidin ;
h. contacting and incubating said immobilized complex of step (g) with a substrate solution wherein said enzyme linked to avidin induces a reaction with said substrate and produces a detectable reaction product on the immobilized complex; and
i. correlating said detectable reaction product on the immobilized complex to the presence of said antigen to be detected.

20. The method of claim 19, wherein after said enzyme-linked avidin is contacted and incubated with said immobilized complex of step (e), said support is washed.

21. The method of claim 19, wherein the anti-antigen antibody of step (b) and the anti-antigen antibody of step (d) are specific for different epitopes of said antigen.

22. The method of claim 19, wherein the biological test fluid lacks endogenous antibodies against said antigen.

23. The method of claim 22, wherein the biological test fluid is a plant extract containing antigens of a plant pathogen.

24. The method fo claim 23, wherein the antigen is a bacterium.

25. The method of claim 24, wherein the bacterium is *Corynebacterium sepedonicum*.

26. A kit for detection of an antibody of interest in a body fluid containing said antibody of interest and other antibodies, comprising:
an antibody-binding protein covalently bound to a support;
a biotinylated antigen specific to said antibody of interest;
enzyme-linked avidin solution; and
enzyme substrate.

27. A kit for detecting an antigen of interest in a body fluid containing said antigen of interest and other antigens, comprising:
an Fc receptor antibody-binding protein covalently bound to a support;
a biotinylated monoclonal antibody modified to be an Fab fragment, lacking the Fc portion which binds to the protein but retaining the ability to bind said antigen of interest;
enzyme-linked avidin solution;
and enzyme substrate.

## Patentansprüche

1. Verfahren zur Bestimmung eines interessierenden Antikörpers in einem den interessierenden Antikörper und andere Antikörper enthaltenden Körperfluid, mit den Schritten:
a. Bereitstellen eines kovalent an einen Träger gebundenen, Antikörper-bindenden Proteins;
b. Kontaktieren und Inkubieren eines Testkörperfluids, enthaltend den interessierenden Antikörper und andere Antikörper, mit dem proteingebundenen Träger gemäß Schritt (a), wobei das Protein mit den Antikörpern unter Immobilisierung der Antikörper einschließlich des interessierenden Antikörpers reagiert;
c. Bereitstellen einer Lösung eines gegen den interessierenden Antikörper spezifischen Antigens, wobei das Antigen biotinyliert ist;
d. Kontaktieren und Inkubieren des biotinylierten Antigens gemäß Schritt (c) mit dem immobilisierten, interessierenden Antikörper gemäß Schritt (b), wobei das biotinylierte Antigen mit dem immobilisierten, interessierenden Antikörper unter Immobilisierung des biotinylierten Antigens reagiert;
e. Bereitstellen einer Lösung von Avidin, wobei das Avidin kovalent an ein Enzym gebunden ist;
f. Kontaktieren und Inkubieren der Lösung gemäß Schritt (e) mit dem immobilisierten, biotinylierten, antigenimmobilisierten, Antikörper-Protein-gebundenen Komplex gemäß Schritt (d), wobei das enzymmarkierte Avidin mit dem Komplex unter Immobiliserung des enzymmarkierten Avidins reagiert;
g. Kontaktieren und Inkubieren des immobilisierten Komplexes gemäß Schritt (f) mit einer Substratlösung, wobei das an Avidin gebundene Enzym eine Reaktion mit dem Substrat induziert und ein bestimmbares Reaktionsprodukt auf dem immobilisiertem Komplex erzeugt; und
h. Korrelieren des bestimmbaren Reaktionsproduktes auf dem immobilisiertem Komplex mit der Anwesenheit des zu bestimmenden, interessierenden Antikörpers.

2. Verfahren nach Anspruch 1, bei dem das Körperfluid Speichel ist.

3. Verfahren nach Anspruch 1, bei dem der Träger ein beliebiges poröses Material mit einer niedrigen nicht-spezifischen Bindung von Proteien und funktionellen Gruppen oder anderen chemischen Gegebenheiten, die die Bindung von antikörperbindenden Proteinen gestatten, ist.

4. Verfahren nach Anspruch 3, bei dem der Träger Agarose ist.

5. Verfahren nach Anspruch 4, bei dem man Farbvergleichskontrollen unter Verwendung zusätzlicher Aliquots an Agarose, voneinander getrennt mit Polyethylenscheiben, laufen läßt.

6. Verfahren nach Anspruch 1, bei dem das Protein Protein A ist.

7. Verfahren nach Anspruch 1, bei dem das Protein von Quellen abgeleitet ist, die aus der von den Typen II, III, IV, V und VI Fc-Rezeptoren gebildeten Gruppe ausgewählt sind.

8. Verfahren nach Anspruch 1, bei dem nach dem Kontaktieren und Inkubieren des Testfluids mit dem proteingebundenen Träger der Träger gewaschen wird.

9. Verfahren nach Anspruch 1, bei dem nach dem Kontaktieren und Inkubieren des biotinylierten Antigens mit dem immobilisiertem Antikörper der Träger gewaschen wird.

10. Verfahren nach Anspruch 1, bei dem nach dem Kontaktieren und Inkubieren des enzymgebundenen Avidins mit dem immobilisierten Antigenkomplex der Träger gewaschen wird.

11. Verfahren nach Anspruch 1, bei dem der Träger in einer offenendigen Kolonne festgehalten wird.

12. Verfahren nach Anspruch 11, bei dem die Kolonne aus Polystyrol besteht.

13. Verfahren nach Anspruch 11, bei dem der Träger von einer porösen Polyethylenscheibe gehalten wird.

14. Verfahren nach Anspruch 1, bei dem die Testkörperflüssigkeit vor dem Auftragen mit einem wäßrigen Puffer verdünnt wird.

15. Verfahren zum Bestimmen eines interessierenden Antikörpers in einem den interessierenden Antikörper und andere Antikörper enthaltenden Körperfluid, mit den Schritten:
a. Bereitstellen eines kovalent an einen Träger gebundenen, antikörperbindenden Proteins,
b. Kontaktieren und Inkubieren eines Testkörperfluids, enthaltend den interessierenden Antikörper und andere Antikörper, mit einer Lösung eines für den interessierenden Antikörper spezifischen Antigens, wobei das Antigen biotinyliert ist,
c. Kontaktieren und Inkubieren der kombinierten Lösung gemäß Schritt (b) mit einer Lösung von Avidin, wobei das Avidin kovalent an ein Enzym gebunden ist,
d. Kontaktieren und Inkubieren der kombinierten Lösung gemäß Schritt (c) mit einem Träger gemäß Schritt (a), so daß ein Komplex von Antikörper-biotinyliertem, Antigen-Enzym-markiertem Avidin durch den Träger immobilisiert wird,
e. Kontaktieren und Inkubieren des immobilisierten Komplexes gemäß Schritt (d) mit einer Substratlösung, wobei das mit Avidin verknüpfte Enzym eine Reaktion mit dem Substrat induziert und ein bestimmbares Reaktionsprodukt auf dem immobilisiertem Komplex bildet; und
f. Korrelieren des bestimmbaren Reaktionsproduktes auf dem immobiliserten Komplex mit der Anwesenheit des zu bestimmenden Antikörpers.

16. Verfahren nach Anspruch 15, bei dem nach dem Kontaktieren und Inkubieren der kombinierten Lösung gemäß Schritt (c) mit dem Träger gemäß Schritt (a) der Träger gewaschen wird.

17. Verfahren nach Anspruch 15, bei dem das Antigen HIV-1 ist.

18. Verfahren nach Anspruch 1 oder 15, bei dem der Assay in einer Pipettespitze durchgeführt wird.

19. Verfahren zum Bestimmen eines Antigens in einem das Antigen enthaltenden biologischen Testfluid, mit den Schritten:
a. Bereitstellen eines Fc-Rezeptor-, Antikörper-bindenden Proteins, das kovalent an einen Träger gebunden ist;
b. Bereitstellen einer das Antigen enthaltenden Lösung des Testfluids, enthaltend eine geringe Menge von Antikörpern gegen das Antigen in einem wäßrigen Puffer;
c. Kontaktieren und Inkubieren der Lösung gemäß Schritt (b) mit dem Träger gemäß Schritt (a), so daß ein Komplex aus Antikörper und Antigen durch den Träger immobilisert wird;
d. Bereitstellen einer Lösung eines monoklonalen Antikörpers, der so modifiziert ist, daß er ein Fab-Fragment ist, dem der Fc-Bereich fehlt, welcher an das Protein bindet, jedoch seine Fähigkeit zur Bindung des Antigens beibehält, wobei der Antikörper biotinyliert ist;
e. Kontaktieren und Inkubieren der Lösung gemäß Schritt (d) mit dem immobilisiertem Komplex gemäß Schritt (c), so daß ein Antikörper-Antigen-Fab-Antikörperkomplex durch den Träger immobilisiert wird;
f. Bereitstellen einer Lösung von Avidin, wobei das Avidin kovalent an ein Enzym gebunden ist;
g. Kontaktieren und Inkubieren der Lösung gemäß Stufe (f) mit dem immobilisierten Antikörper-Antigen-Fab-Antikörperkomplex gemäß Stufe (e), wobei das enzymmarkierte Avidin mit dem Komplex unter Immobilisierung des enzymmarkierten Avidins reagiert;
h. Kontaktieren und Inkubieren des immobilisierten Komplexes gemäß Stufe (g) mit einer Substratlösung, wobei das mit Avidin verbundene Enzym eine Reaktion mit dem Substrat induziert und ein bestimmbares Reaktionsprodukt auf dem immobilisierten Komplex bildet; und
i. Korrelieren des bestimmbaren Reaktionsproduktes auf dem immobilisiertem Komplex mit der Anwesenheit des zu bestimmenden Antigens.

20. Verfahren nach Anspruch 19, bei dem nach dem Kontaktieren und Inkubieren des enzymgebundenen Avidins der Träger gewaschen wird.

21. Verfahren nach Anspruch 19, bei dem der anti-Antigen-Antikörper gemäß Schritt (b) und der anti-Antigen-Antikörper gemäß Schritt (d) für verschiedene Epitope des Antigens spezifisch sind.

22. Verfahren nach Anspruch 19, bei dem der biologischen Testflüssigkeit endogene Antikörper gegen das Antigen fehlen.

23. Verfahren nach Anspruch 22, bei dem das biologische Testfluid ein Pflanzenextrakt ist, der Antigene eines Pflanzenpathogens enthält.

24. Verfahren nach Anspruch 23, bei dem das Antigen ein Bakterium ist.

25. Verfahren nach Anspruch 24, bei dem das Bakterium Coryne-bacterium sepedonicum ist.

26. Kit für die Bestimmung eines interessierenden Antikörpers in einem den interessierenden Antikörper und andere Antikörper enthaltenden Körperfluid, enthaltend:
ein antikörperbindendes Protein, das kovalent an einen Träger gebunden ist;
ein biotinyliertes Antigen, das gegen den interessierenden Antikörper spezifisch ist;
eine Lösung von enzymverknüpftem Avidin; und
ein Enzymsubstrat.

27. Kit für die Bestimmung eines interessierenden Antigens in einem das interessierende Antigen und andere Antigene enthaltenden Körperfluid, enthaltend:
ein Fc-Rezeptor-, antikörperbindendes Protein, das kovalent an einen Träger gebunden ist;
einen biotinylierten monoklonalen Antikörper, der zu einem Fab-Fragment modifiziert ist, dem der Fc-Bereich, der an das Protein bindet, fehlt, der jedoch die Fähigkeit zur Bindung des interessierenden Antigens beibehalten hat;
eine Lösung von enzymgebundenem Avidin; und
ein Enzymsubstrat.

## Revendications

1. Procédé pour détecter un anticorps particulier, qui présente un intérêt, dans un fluide corporel contenant ledit anticorps particulier et d'autres anticorps, ledit procédé comprenant les étapes consistant à :
a. faire appel à une protéine fixant les anticorps et liée par covalence à un support ;
b. mettre en contact et incuber un fluide corporel d'essai contenant ledit anticorps particulier et d'autres anticorps avec ladite protéine liée au support de l'étape (a), de façon que ladite protéine réagisse avec lesdits anticorps pour immobiliser lesdits anticorps, y compris ledit anticorps particulier ;
c. faire appel à une solution d'un antigène spécifique dudit anticorps particulier, ledit antigène étant biotinylé ;
d. mettre en contact et incuber ledit antigène biotinylé de l'étape (c) avec l'anticorps particulier immobilisé de l'étape (b), de façon que ledit antigène biotinylé réagisse avec ledit anticorps particulier immobilisé pour immobiliser ledit antigène biotinylé ;
e. faire appel à une solution d'avidine, ladite avidine étant couplée par covalence à un enzyme ;
f. mettre en contact et incuber ladite solution de l'étape (e) avec le complexe immobilisé protéine liée-anticorps-antigène biotinylé de l'étape (d), de façon que ladite avidine couplée à l'enzyme réagisse avec ledit complexe pour immobiliser ladite avidine couplée à l'enzyme ;
g. mettre en contact et incuber le complexe immobilisé de l'étape (f) avec une solution d'un substrat, de façon que ledit enzyme couplé à l'avidine induise une réaction avec ledit substrat et fournisse un produit de réaction détectable sur le complexe immobilisé ; et,
h. déduire dudit produit de réaction détectable sur le complexe immobilisé la présence dudit anticorps particulier à détecter.

2. Procédé suivant la revendication 1, dans lequel le fluide corporel est la salive.

3. Procédé suivant la revendication 1, dans lequel ledit support est un matériau poreux ayant un faible pouvoir de fixer de façon non-spécifique les protéines et les groupes fonctionnels ou autres réactifs chimiques qui permettent la fixation de protéines qui se lient aux anticorps.

4. Procédé suivant la revendication 3, dans lequel ledit support est l'agarose.

5. Procédé suivant la revendication 4, dans lequel les essais témoins pour comparaison colorée sont mis en oeuvre en utilisant des parties aliquotes supplémentaires d'agarose séparées par des disques de polyéthylène.

6. Procédé suivant la revendication 1, dans lequel ladite protéine est la protéine A.

7. Procédé suivant la revendication 1, dans lequel ladite protéine provient de sources choisies parmi l'ensemble constitué par les récepteurs de Fc des types II, III, IV, V et VI.

8. Procédé suivant la revendication 1, dans lequel, après mise en contact et incubation du fluide corporel d'essai avec ladite protéine liée à son support, on lave ledit support.

9. Procédé suivant la revendication 1, dans lequel, après mise en contact et incubation dudit antigène biotinylé avec ledit anticorps immobilisé, on lave ledit support.

10. Procédé suivant la revendication 1, dans lequel, après mise en contact et incubation dudit enzyme couplé à l'avidine avec ledit complexe d'antigène immobilisé, on lave ledit support.

11. Procédé suivant la revendication 1, dans lequel ledit support est piégé dans une colonne à extrémités ouvertes.

12. Procédé suivant la revendication 11, dans lequel ladite colonne est en polystyrène.

13. Procédé suivant la revendication 11, dans lequel ledit support est porté par un disque poreux en polyéthylène.

14. Procédé suivant la revendication 1, dans lequel ledit fluide corporel d'essai est dilué avant utilisation au moyen d'un tampon aqueux.

15. Procédé pour détecter un anticorps particulier, qui présente un intérêt, dans un fluide corporel contenant ledit anticorps particulier et d'autres anticorps, ledit procédé comprenant les étapes consistant à :
a. faire appel à une protéine fixant les anticorps et liée par covalence à un support ;
b. mettre en contact et incuber un fluide corporel d'essai contenant ledit anticorps particulier et d'autres anticorps avec une solution d'un antigène spécifique dudit anticorps particulier, ledit antigène étant biotinylé ;
c. mettre en contact et incuber ladite solution résultant de l'étape (b) avec une solution d'avidine, ladite avidine étant couplée par covalence à un enzyme ;
d. mettre en contact et incuber ladite solution résultant de l'étape (c) avec un support selon l'étape (a), de telle façon qu'un complexe anticorps-antigène biotinylé-avidine couplée à l'enzyme soit immobilisé par ledit support ;
e. mettre en contact et incuber le complexe immobilisé de l'étape (d) avec une solution d'un substrat, de façon que ledit enzyme couplé à l'avidine induise une réaction avec ledit substrat et fournisse un produit de réaction détectable sur le complexe immobilisé ; et,
f. déduire dudit produit de réaction détectable sur le complexe immobilisé la présence dudit anticorps particulier à détecter.

16. Procédé suivant la revendication 15, dans lequel, après mise en contact et incubation de la solution résultante de l'étape (c) avec le support de l'étape (a), on lave ledit support.

17. Procédé suivant la revendication 15, dans lequel l'antigène est HIV-1.

18. Procédé suivant la revendication 1 ou 15, dans lequel l'essai est effectué dans une pipette.

19. Procédé pour détecter un antigène dans un fluide corporel d'essai contenant ledit antigène, ledit procédé comprenant les étapes consistant à :
a. faire appel à une protéine du type récepteur de Fc, fixant les anticorps et liée par covalence à un support ;
b. faire appel à une solution d'un fluide d'essai contenant ledit antigène et une faible quantité d'un anticorps dirigé contre ledit antigène dans un tampon aqueux ;
c. mettre en contact et incuber la solution de l'étape (b) avec le support de l'étape (a), de telle façon qu'un complexe de l'anticorps et de l'antigène soit immobilisé par ledit support ;
d. faire appel à une solution d'un anticorps monoclonal modifié de façon qu'il soit un fragment Fab (i) dépourvu de la portion Fc qui se fixe sur la protéine mais (ii) conservant la capacité de se lier audit antigène, ledit anticorps étant biotinylé ;
e. mettre en contact et incuber ladite solution de l'étape (d) avec le complexe immobilisé de l'étape (c), de telle façon qu'un complexe anticorps-antigène-anticorps Fab soit immobilisé par ledit support ;
f. faire appel à une solution d'avidine, ladite avidine étant couplée par covalence à un enzyme ;
g. mettre en contact et incuber ladite solution de l'étape (f) avec ledit complexe anticorps-antigène-anticorps Fab immobilisé de l'étape (e), de telle façon que ladite avidine couplée à l'enzyme réagisse avec ledit complexe pour immobiliser ladite avidine couplée à l'enzyme ;
h. mettre en contact et incuber ledit complexe immobilisé de l'étape (g) avec une solution d'un substrat, de façon que ledit enzyme couplé à l'avidine induise une réaction avec ledit substrat et fournisse un produit de réaction détectable sur le complexe immobilisé ;
i. déduire dudit produit de réaction détectable sur le complexe immobilisé la présence dudit antigène à détecter.

20. Procédé suivant la revendication 19, dans lequel, après mise en contact et incubation de ladite avidine couplée à l'enzyme avec ledit complexe immobilisé de l'étape (e), on lave ledit support.

21. Procédé suivant la revendication 19, dans lequel l'anticorps anti-antigène de l'étape (b) et l'anticorps anti-antigène de l'étape (d) sont spécifiques d'épitopes différents dudit antigène.

22. Procédé suivant la revendication 19, dans lequel le fluide biologique d'essai est dépourvu d'anticorps endogènes dirigés contre ledit antigène.

23. Procédé suivant la revendication 22, dans lequel le fluide biologique d'essai est un extrait végétal contenant des antigènes d'un pathogène de plante.

24. Procédé suivant la revendication 23, dans lequel l'antigène est un bacterium.

25. Procédé suivant la revendication 24, dans lequel le bacterium est le ***corynebacterium sepedonicum***.

26. Nécessaire pour la détection d'un anticorps particulier, qui présente un intérêt, dans un fluide corporel contenant ledit anticorps particulier et d'autres anticorps, ledit nécessaire comprenant :
- une protéine fixant les anticorps et liée par covalence à un support ;
- un antigène biotinylé spécifique dudit anticorps particulier ;
- une solution d'avidine couplée à un enzyme ; et,
- un substrat de l'enzyme.

27. Nécessaire pour la détection d'un antigène particulier, qui présente un intérêt, dans un fluide corporel contenant ledit antigène particulier et d'autres antigènes, ledit nécessaire comprenant :
- une protéine du type récepteur de Fc, fixant les anticorps, et liée par covalence à un support ;
- un anticorps monoclonal modifié pour être un fragment Fab (i) dépourvu de la portion Fc qui se fixe sur la protéine mais (ii) conservant la capacité de se lier audit antigène particulier ;
- une solution d'avidine couplée à un enzyme ; et,
- un substrat de l'enzyme.
